# EUROPEAN PATENT SPECIFICATION

(11) **EP 3 911 147 B1**
(45) Date of publication and mention of the grant of the patent: **16.07.2025**
(21) Application number: 19880924.6
(22) Date of filing: 09.12.2019
(51) Int. Cl.: A01H 5/08, A01H 6/82, C07K 14/415, C12Q 1/6895

(54) **TOMATO PLANT RESISTANT TO TOMATO BROWN RUGOSE FRUIT VIRUS**
GEGEN JORDAN-VIRUS RESISTENTE TOMATENPFLANZE
PLANT DE TOMATE RÉSISTANT AU VIRUS DU FRUIT RUGUEUX BRUN DE TOMATE

(30) Priority: 14.01.2019 WO PCT/EP2019/050830
(43) Date of publication of application: 24.11.2021
(73) Proprietor: Enza Zaden Beheer B.V., 1602 DB Enkhuizen (NL)
(72) Inventor: YKEMA, Marieke, 1602 DB Enkhuizen (NL); VERWEIJ, Cornelis Walter, 1602 DB Enkhuizen (NL); DE LA FUENTE VAN BENTEM, Sergio, 1602 DB Enkhuizen (NL); PEREFARRES, Frederic Michel Pierre, 1602 DB Enkhuizen (NL)
(74) Representative: Arnold & Siedsma
(86) International application number: PCT/EP2019/084272
(87) International publication number: WO 2020/148021

(56) References cited:
- WO-A1-2018/219941
- WO-A2-2004/020594
- DATABASE EMBL [online] 13 December 2006 (2006-12-13), "Solanum lycopersicum genomic DNA, chromosome 8, clone: C08SLe0082C24.", XP002791884, retrieved from EBI accession no. EM_STD:AP009297 Database accession no. AP009297
- AINONG SHI ET AL: "Molecular Markers for Tm-2 Alleles of Tomato Mosaic Virus Resistance in Tomato", AMERICAN JOURNAL OF PLANT SCIENCES, vol. 02, no. 02, 1 January 2011 (2011-01-01), US, pages 180 - 189, XP055392176, ISSN: 2158-2742, DOI: 10.4236/ajps.2011.22020
- P. KADIRVEL ET AL: "Mapping of QTLs in tomato line FLA456 associated with resistance to a virus causing tomato yellow leaf curl disease", EUPHYTICA, vol. 190, no. 2, 5 December 2012 (2012-12-05), NL, pages 297 - 308, XP055392076, ISSN: 0014-2336, DOI: 10.1007/s10681-012-0848-0
- SALEM N ET AL: "A new tobamovirus infecting tomato crops in Jordan", ARCHIVES OF VIROLOGY, SPRINGER WIEN, AT, vol. 161, no. 2, 19 November 2015 (2015-11-19), pages 503 - 506, XP035888535, ISSN: 0304-8608, [retrieved on 20151119], DOI: 10.1007/S00705-015-2677-7
- NETA LURIA ET AL: "A New Israeli Tobamovirus Isolate Infects Tomato Plants Harboring Tm-22 Resistance Genes", PLOS ONE, vol. 12, no. 1, 20 January 2017 (2017-01-20), pages e0170429, XP055469071, DOI: 10.1371/journal.pone.0170429
- MAAYAN YONATAN ET AL: "Using genomic analysis to identify tomatoTm-2resistance-breaking mutations and their underlying evolutionary path in a new and emerging tobamovirus", ARCHIVES OF VIROLOGY, SPRINGER WIEN, AT, vol. 163, no. 7, 27 March 2018 (2018-03-27), pages 1863 - 1875, XP036525397, ISSN: 0304-8608, [retrieved on 20180327], DOI: 10.1007/S00705-018-3819-5

## Description

Disclosed herein is a plant of the *S*. *lycopersicum* species that is resistant to Tobamovirus, wherein the plant comprises one or more genomic sequences. More specifically tomato plants (*S. lycopersicum*) that are resistant to Tomato Brown Rugose Fruit Virus (TBRFV). The present invention relates to a resistance gene or a genomic sequence providing resistance to Tobamovirus, wherein the Tobamovirus is TBRFV. Furthermore, the present invention relates to methods for providing a *S*. *lycopersicum* plant that is resistant to Tobamovirus.

Tobamovirus is a genus in the virus species Virgaviridae that infects plants, including plants of the *Solanaceae* family, such as tobacco, potato, tomato, and eggplant and are among the most serious threats to vegetable crops in the world. Tobamoviruses are particularly a problem in tomato crops grown in protected environments and are transmitted over long distances through external seed contamination, and mechanically from plant to plant through common culture practices through workers' hands, clothes, tools, and are capable to preserve infectivity in seeds and contaminated soil. Furthermore, common weeds, often asymptomatic when infected by the virus comprise a cryptic reservoir between growth cycles.

Tobamovirus infections can have disastrous effects in crops when they become contaminated. Prevention of infection, by, for example, raising seedlings in a virus free environment is generally costly and/or unfriendly to the environment. In addition, these methods do not always provide satisfactory results.

Tobamoviruses are non-enveloped, with helical rod geometries, and helical symmetry. Viral particles are rod-shaped and have a diameter of around 18 nm, and a length of 300 to 310 nm. Their positive-sense single stranded RNA genomes are linear and non-segmented, and around 6.3 to 6.5kb in length. There are over 35+ virus species in this genus including Tomato Mosaic Virus (ToMV) or Tobacco Mosaic Virus (TMV), Tomato Mild Mottle Virus (ToMMV), and the recently newly discovered Tomato Brown Rugose Fruit Virus (TBRFV).

In tomato, naming of the four strains of Tobamovirus (more specifically ToMV) currently recognized (Tm-0, Tm-1, Tm-2 and Tm-2²) is based on the introgressed resistance (R) genes *Tm1, Tm2* and *Tm2²* from related wild species. The *Tm1* gene was introgressed from *Solanum habrochaites* and is incompletely dominant. The *Tm2* and *Tm2²* genes were introgressed from *Solanum peruvianum* and confer dominant complete resistance to ToMV. However new strains of Tobamovirus have emerged as resistance is overcome and recently resistance-breaking Tobamo virus species have been reported in commercial fields in Mexico, Jordan, and Israel.

In the end of 2014 and beginning of 2015 an outbreak of a new disease infecting tomatoes occurred in Israel and Jordan. Symptomatic plants showed a mosaic pattern on leaves accompanied occasionally by narrowing of leaves and yellow spotted fruit. Research showed that this new disease was a new Tobamovirus, called TBRFV. TBRFV infection is associated with necrotic lesions on leaves and tomato plants show mild foliar symptoms at the end of the season but strong brown rugose symptoms on fruits, making the fruit unsuitable for consumption. Furthermore, regarding to other members of the *Solanaceae* family, it seems that the TBRFV is capable to infect pepper plants as well, e.g. when planted on contaminated soil from previous growth cycle of infected tomato plants in high temperatures above 30°C.

In the battle against Tobamovirus, resistance was introduced in tomatoes by introgression of the R genes *Tm2* and *Tm2²,* resulting in resistance to ToMV. However, these R-genes do not provide resistance to the new TBRFV, since different domains in the viral proteins comprised of different protein structure and a new resistance mechanism and/or resistant genes are required for a different resistance mechanism. Furthermore, it is highly likely that over time resistance will be broken, since the virus will adapt and evolve resulting in viral breakthrough. Therefore, new resistance genes need to be identified and/or combined to provide resistant crops, especially against the new TBRFV.

WO2018/219941A1 disclosed tolerance to the tobamovirus TBRFV in plants of solanum lycopersicum comprising in its genome QTLs characterized by defined alleles of different SNPs on chromosome 6, 9 and 11 and confering to the plant an improved phenotype corresponding to foliar and/or fruit tolerance against TBRFV.

Considering the above, there is a need in the art for TBRFV resistant tomato plants, more specifically TBRFV resistant S. *lycopersicum.* In addition, there is a need in the art to provide methods and means for providing TBRFV resistant S. *lycopersicum* plants.

It is an object of the present invention, amongst other objects, to address the above need in the art. The object of present invention, amongst other objects, is met by the present invention as outlined in the appended claims.

Specifically, the above object, amongst other objects, is met, according to a first aspect, by claim 1. It is predicted that the TBRFV resistance gene encodes for a NBS-LRR resistance protein.

The present invention relates to wherein the TBRFV resistance gene comprising a coding sequence that has at least 95%, preferably at least 98%, more preferably at least 99%, most preferably 100% nucleotide sequence identity with SEQ ID No.115. Disclosed herein is a plant, wherein the plant comprises one or more genomic sequences selected from the group consisting of SEQ ID No.1, SEQ ID No.2 SEQ ID No.3, SEQ ID No.4, SEQ ID No.5, SEQ ID No.6, SEQ ID No.7, SEQ ID No.8, SEQ ID No.9, SEQ ID No.10, SEQ ID No.11, SEQ ID No.12, SEQ ID No.13, SEQ ID No.14, SEQ ID No.15, SEQ ID No.16, SEQ ID No.17 and SEQ ID No.18, or having at least 95% sequence identity with any of said SEQ ID No's. The genomic sequences encode for one or more genes or genetic elements that provide resistance to Tobamovirus. Sequences have been examined on gene homology using public database of the National Center for Biotechnology Information (NCBI). Six genomic sequences have homology with sequences that encode for NBS-LRR resistance proteins (SEQ ID No.7, 8, 9, 10, 11 and 14). Four genomic sequences have homology with LRR receptor-like serine/threonine-protein kinase (SEQ ID No. 5, 6, 12 and 13).

Pathogen recognition by plants takes place via two relevant groups of host receptors involving two main types of proteins, namely Receptor-like kinases or proteins (RLK or RLP) and nucleotide-binding site leucine-rich repeat proteins (NBS-LRR resistance proteins). The first group are pattern recognition receptors (PRR) specializing in the recognition of pathogen associated molecular patterns (PAMPS). RLPs or RLKs are attached to the cell membrane and are extracellular immune receptors. Plant RLKs are involved in plant-pathogen interaction and defence responses and plant receptor kinases (PRKs) can be defined as proteins that contain an extracellular domain, a single-pass transmembrane domain and a cytoplasmic serine/threonine (ser/thr) protein kinase domain. Plant LRR-RLKs (leucine rich-repeat receptor-like kinase) possess a functional cytoplasmic kinase domain, and all of the plant LRR-RLKs analysed to date possess ser/thr kinase activity. The resistance to pathogens provided by these receptors is called PAMP-triggered immunity (PTI). The other group mainly comprises intracellular receptors called resistance proteins (R proteins). The majority of disease resistance genes in plants encode nucleotide-binding site leucine-rich repeat proteins, also known as NBS-LRR proteins. These proteins are characterized by nucleotide-binding site (NBS) and leucine-rich repeat (LRR) domains as well as variable amino- and carboxy-terminal domains and are involved in the detection of diverse pathogens, including bacteria, viruses, fungi, nematodes, insects and oomycetes. The majority of the identified genomic sequences that provide Tobamovirus resistance comprise multiple LRR domains. It is thought that these domains determine effector recognition and therefore disease susceptibility/resistance.

Pathogens develop counter strategies to overcome PTI through modifying or changing PAMPs or MAMPs. Then, plants will develop a way to recognize these effectors and trigger a faster and stronger secondary defence response known as effector-triggered immunity (ETI). ETI is mediated by R proteins and accompanied by localized cell death around the site of infection. The presence of these newly identified resistance gene and/or genomic regions encoding NBS-LRR proteins and/or plant receptor kinases will decrease the chances of the pathogen overcoming the resistance, or when combined with other resistance genes, disease resistance may even be further improved. Disclosed herein is a plant, wherein the plant comprises the genomic sequence represented by SEQ ID No.3. The genomic sequence SEQ ID No. 3 comprises multiple sequences that have homology with sequences that encode for NBS-LRR resistance proteins and LRR receptor-like serine/threonine-protein kinase.

Disclosed herein is a plant, wherein the plant comprises SEQ ID No.8, SEQ ID No.9, SEQ ID No. 10 and SEQ ID No.11.

According to present invention, Tobamovirus resistance of the plant may be affected by a sequence encoding a NBS-LRR protein

Disclosed herein is a plant, wherein the plant comprises the genomic sequences of SEQ ID No.8, SEQ ID No.11 and SEQ ID No.14.

Disclosed herein is a plant, wherein the plant comprises SEQ ID No.8, SEQ ID No.9, SEQ ID No.10, SEQ ID No.11, SEQ ID No.12, SEQ ID No.13 and SEQ ID No.14.

Disclosed herein is a plant, wherein the plant is resistant to Tobamo virus strains Tm-0, Tm-1 and Tm-2. In tomato, four strains of Tobamovirus have been identified; Tm-0, Tm-1, Tm-2 and Tm-2².

Disclosed herein is a plant, wherein the plant is resistant to Tomato Brown Rugose Fruit Virus (TBRFV).

Disclosed herein is a plant, wherein the TBRFV is virus isolate AE050.

Disclosed herein is a plant, wherein the plant is a tomato plant (*Solanum lycopersicum*).

Disclosed herein is a plant, wherein the one or more genomic sequences and/or TBRFV resistance gene is heterozygous or homozygous present in the genome of said plant. From the experimental data it can be concluded that the resistance is dominant and that the TBRFV resistance gene and/or genomic sequence must be at least heterozygous present in the genome of the plant to provide resistance against the Tobamo virus.

Disclosed herein is a plant, wherein said TBRFV resistance gene and/or one or more genomic sequences are as found in the deposit accession number NCIMB 43279. NCIMB 43279, *Solanum lycopersicum* seeds were deposited on 16 November 2018 at NCIMB Ltd, Ferguson Building, Craibstone Estate Bucksburn, AB21 9YA Aberdeen, United Kingdom.

The present invention relates to a resistance gene (TBRFV resistance gene) for providing resistance to a Tobamovirus in a *S*. *lycopersicum* plant, wherein said resistance gene is represented by a coding sequence having at least 95% nucleotide sequence identity with SEQ ID No.115, wherein the Tobamovirus is TBRFV.

The present invention, according to a further aspect, relates to a genomic sequence for providing resistance to a Tobamovirus in a *S*. *lycopersicum* plant, wherein the genomic sequence is SEQ ID No.14, or having at least 95% sequence identity with SEQ ID No.14, wherein the Tobamovirus is TBRFV.

Disclosed herein is a a resistance locus for providing resistance to a Tobamovirus in a *S*. *lycopersicum* plant, wherein the locus is represented by SEQ ID No.1, SEQ ID No.2, SEQ ID No.3 or SEQ ID No.4, preferably SEQ ID No.3.

According to the present invention, the resistance gene or genomic sequence provides resistance to a TBRFV.

Disclosed herein is a method for providing a plant of the *S*. *lycopersicum* species that is resistant to Tobamovirus, wherein the method comprises the steps of;
a) selecting a *S*. *habrochaites* plant that is resistant to Tobamovirus, wherein said selection comprises establishing the presence of the resistance gene or genomic sequence of present invention,
b) transferring the identified genomic sequence or locus of step a) into a *S*. *lycopersicum* plant thereby conferring Tobamovirus resistance to said *S*. *lycopersicum* plant.
Transferring can be done by crossing the selected *S*. *habrochaites* plant with a *S*. *lycopersicum.* Subsequently, a Tobamovirus resistant *S*. *lycopersicum* plant can be selected.

According to another preferred embodiment, the present invention relates to the method of claim 3, selecting *S*. *lycopersicum* plants that are resistant to Tobamovirus.

According to a preferred embodiment, the present invention relates to the method, wherein establishing the presence of the resistance gene (TBRFV resistance gene) or resistance conferring genomic sequence is performed by one or more markers selected from the group consisting of SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, and SEQ ID No: 94, SEQ ID No. 105, SEQ ID No. 106, SEQ ID No. 107, SEQ ID No. 108, SEQ ID No. 109, SEQ ID No. 110, SEQ ID No. 111, and SEQ ID No. 112, preferably SEQ ID No. 105, SEQ ID No. 106, SEQ ID No. 107, SEQ ID No. 108, SEQ ID No. 109, SEQ ID No. 110, SEQ ID No. 111, and SEQ ID No. 112.

The present invention, according to a further aspect, relates to a use of a marker for establishing the presence of the TBRFV resistance gene or the TBRFV resistance conferring genomic sequence in a *S*. *lycopersicum* plant, wherein the marker is one or more selected from the group consisting of SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, and SEQ ID No: 94, SEQ ID No. 105, SEQ ID No. 106, SEQ ID No. 107, SEQ ID No. 108, SEQ ID No. 109, SEQ ID No. 110, SEQ ID No. 111, and SEQ ID No. 112, preferably SEQ ID No. 105, SEQ ID No. 106, SEQ ID No. 107, SEQ ID No. 108, SEQ ID No. 109, SEQ ID No. 110, SEQ ID No. 111, and SEQ ID No. 112.

The present invention will be further detailed in the following examples and figures wherein:

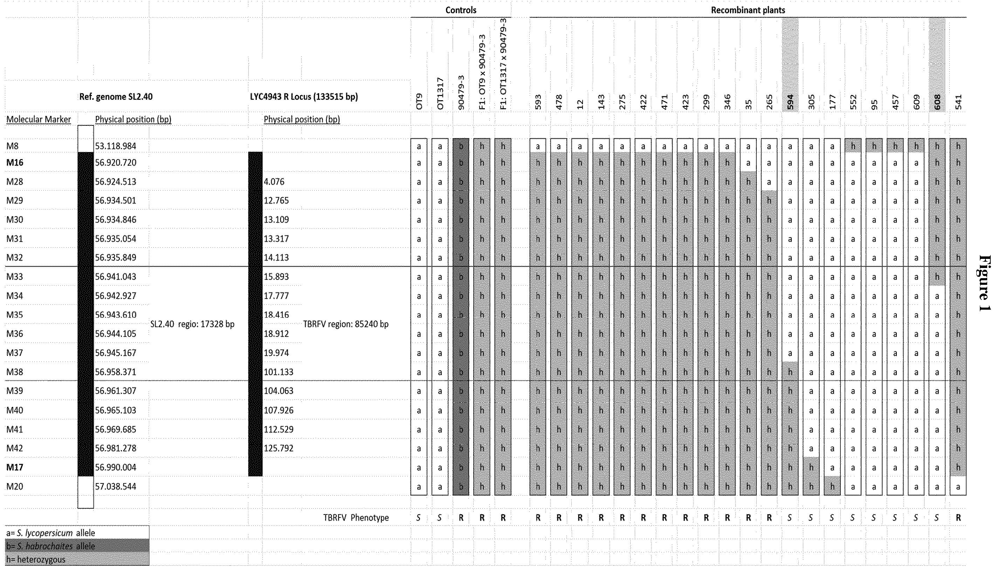

- **Figure 1:**: Overview of the mapping of the locus providing resistance against Tobamovirus, more specifically TRBFV in *S*. *lycopersicum.* F1 plants were created by crossing *S. habrochaites* line 90479-3 that was selected for resistance phenotype with *S. lycopersicum* lines OT9 and OT1317 to create two populations for mapping. Over 700 plants were tested on TRBFV resistance and several recombinant plants (21 plants) were selected and the results of the disease test are combined with the marker data. Several molecular markers (M1 to M42) have been used to determine the position and size of the genomic sequence providing resistance to TRBFV. A clear segregation was observed between resistant (R) and susceptible plants (S). The results indicated that a genomic region located between markers M16 and M17 was providing the TRBFV resistance; the corresponding locus (named LYC4943 R Locus) is 133.515 bp in length and comprises several putative genes. Based on further fine mapping, the size and location of the genomic sequence that is harbouring the TBRFV resistance is determined to be between markers M33 and M38 and is approximately 68.000 bp larger compared to the SL2.40 reference genome of *S. lycopersicum* (85.240 bp vs. 17.328 bp, respectively). It is therefore most likely that one or more genes are located within this region, indicated as "TBRFV region", is providing the TBRFV resistance.

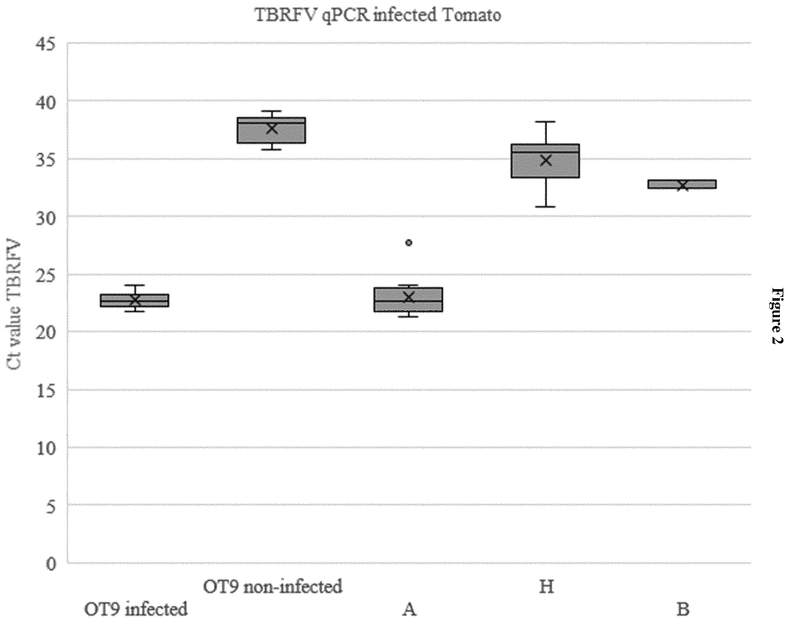

- **Figure 2:**: shows the results of a qPCR for the detection of TBRFV in infected and uninfected tomato plants (*S*. *lycopersicum)* and plants that do not comprise the TBRFV resistance locus. Low Ct values (i.e. below 30) indicate the high presence of viral RNA present in the samples. OT9 is a tomato plant that does not comprise the TBRFV resistance locus. The control sample (OT9 uninfected) showed a Ct value of between 35 and 40 cycles and the infected control sample (OT9 infected) showed a Ct value of between 20 and 25. Plants that show a Ct value above 30 cycles, preferably around 35 cycles were considered resistant, whereas plants that show a Ct value below 30 were considered susceptible. Tomato plants comprising the TBRFV resistance locus, homozygous (B) or heterozygous (H) have a Ct value above 30 cycles and are considered being resistant. Furthermore, the results are showing that the resistance is dominant. Plants that did not comprise the TBRFV resistance locus (A and OT9 infected) showed a Ct value of between 20 and 25, indicating that the plant was susceptible to TBRFV infection.

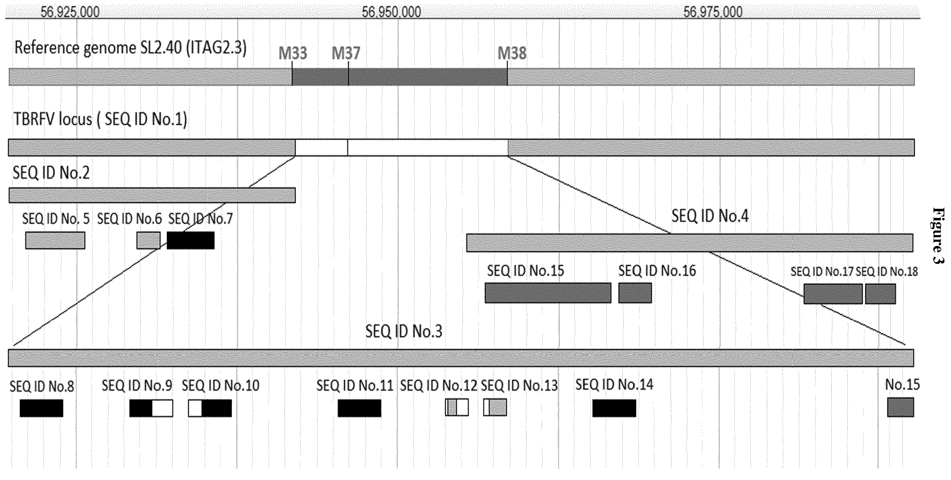

- **Figure 3:**: shows a schematic overview of the genomic sequences SEQ ID No.1 to SEQ ID No.18 that encode for one or more genes or genetic elements that provide resistance to Tobamovirus.

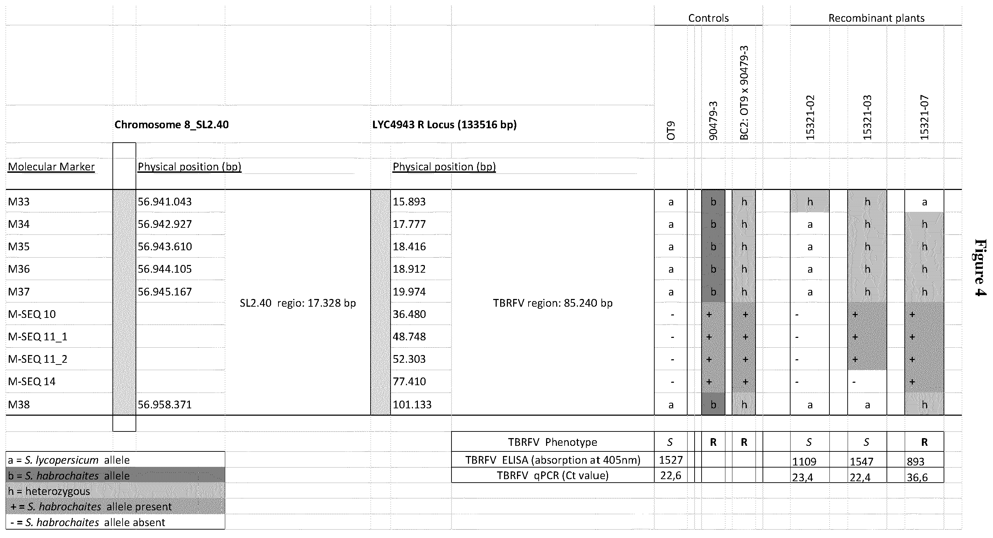

- **Figure 4:**: Overview of a further mapping of the locus providing resistance against Tobamovirus in addition to figure 1. A further recombinant selection has been performed by further genotyping plants with M33 and M38 to identify recombinant plants in the identified TBRFV region and further limit this TBRFV region. Recombinant plants have been further genotyped with markers (M-SEQ 10, M-SEQ 11-1, M-SEQ 11-2, and M-SEQ 14) covering the TBRFV locus and were specifically designed to eliminate candidate regions in the TBRFV locus, i.e. the genomic sequences SEQ ID No.1 to SEQ ID No.18 that encode for one or more genes or genetic elements that provide resistance to Tobamovirus. Recombinant plants 15321-02, 15321-03 and 15321-07 were screened for resistance by inoculation with TBRFV isolate AE50. Based on these recombinant plants in combination with phenotyping, ELISA and qPCR data for the determination of TBRFV infection, it was concluded that the gene conferring resistance is part of genomic SEQ ID No 14. Plants 15321-02 and 15321-03 do not comprise SEQ ID No 14 and showed to be susceptible to TBRFV, with high ELISA scores and low qPCR ct-values that correspond to the values obtained with the susceptible control line OT9, indicating virus infection. Plant 15321-07 comprises the SEQ ID No 14 and showed to be resistant to TBRFV, with low ELISA scores and high qPCR ct-values.

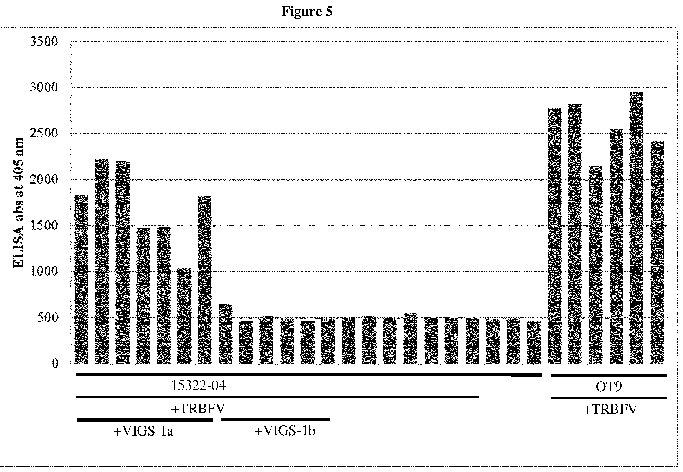

- **Figure 5:**: Shows the TBRFV infection by ELISA in a homozygous TBRFV resistant line (15322-04) as well as a susceptible control line (OT9). Plants were infected with TBRFV (+TRBFV) and infiltrated with construct VIGS-01a that specifically targets the TRBFV resistance gene or with construct VIGS-01b which targets a different region within the identified TRBFV region. ELISA reading was done by measurement of absorption at 405 nm. Control plants OT9 infected by TBRFV resulted in absoption levels of 2000 abs or higher, whereas the resistant plant lines infected with TRBFV resulted in absorption levels of approximately 500 abs. In case the TRBFV resistance gene was silenced by VIGS-01a in the resistant plant lines, absorption levels of between 1500 and 2250 abs were measured, indicating viral infection. Silencing by VIGS-01b in the resistant plant lines, resulted in similar absorption levels as was observed in the infected resistant plant lines that were not silenced by VIGS.

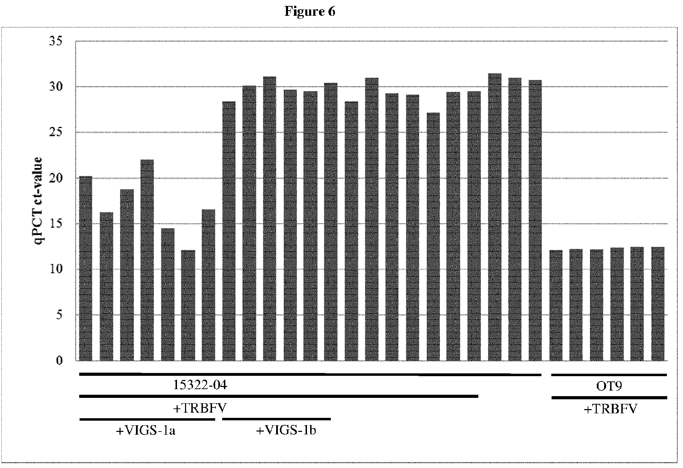

- **Figure 6:**: Shows the TBRFV infection by qPCR in a homozygous TBRFV resistant line (15322-04) as well as a susceptible control line (OT9). Plants were infected with TBRFV (+TRBFV) and infiltrated with construct VIGS-01a that specifically targets the TRBFV resistance gene or with construct VIGS-01b which targets a different region within the identified TRBFV region. The infected control sample showed a Ct value of approximately 12 or 13. The resistant plant lines infected with TRBFV showed a Ct value of approximately 30, indicating TBRFV resistance. In case the TRBFV resistance gene was silenced by VIGS-01a in the resistant plant lines, Ct values were observed to drop to between approximately 12 to 20, higher than the infected control cells, and clearly indicating viral infection. Silencing by VIGS-01b in the resistant plant lines, resulted in similar Ct levels (Ct value of ~30) as was observed in the infected resistant plant lines that were not silenced by VIGS.

### Examples

### Innoculation of a tomato plant with TBRFV

The TBRFV isolate AE050 (Origin Saudi Arabia) was used to perform the disease assays. As plant material, the Line OT9, which is a plant line susceptible for TBRFV was used for virus maintenance. Symptomatic leaves received from the original samples were used for sap-mechanical inoculation on the Line OT9. The virus was maintained on systemically infected tomato plants OT9 by monthly sap-mechanical inoculation on new 3 weeks-old seedlings.

The tomato plants of the species of *Solanum pennellii, S. peruvianum, S. chilense, S. habrochaites, S. pimpinellifolium, S. neorickii, S. corneliomulleri, S. chmielewskii, S. cheesmaniae, S. galapagense* have been screened (~800 out of 912 wild *Solanum* accessions in total). Twelve plants of each accession were infected with TBRFV isolate AE050.

Seeds were sown in vermiculite, seedlings were transplanted in rockwool blocks and inoculated at 4 weeks after sowing. As starting material symptomatic leaves from infected-OT9 were collected and ground in a mortar and pestle in chilled demi water with carborundum (1 gr/100mL). The oldest leaf from 3 weeks-old seedlings of each test plant was mechanically inoculated with AE050 by gently rubbing the leaf once with one finger.

The plants were phenotyped by visual scoring of the plants and the leaves. Plants are scored for visual symptoms at regular time intervals. Symptoms were visually assessed at 2, 4 and 6 weeks after inoculation, and ELISA tests on remaining plants were done from 6 weeks onwards, with 1 month intervals. More than 50% of the plants showed already symptoms at 2 weeks after inoculation.

Visual scoring was performed on a weekly basis. Plants are scored for visual symptoms. The presence of yellowing, mosaic pattern on leaves, leaf deformation (narrowing, mottling) was recorded on a weekly basis at the plant level. First symptoms were typically observed 12-14 days post-inoculation. Plants were categorized as "resistant" when no such symptoms on leaves were observed. Plants displaying any of the symptoms on leaves were categorized as "susceptible". Leaf samples were collected from asymptomatic plants (i.e resistant) to test for the presence of virus either by ELISA.

The screening allowed the selection of several candidates for resistance breeding, with the best candidate being LYC4943, a *S*. *habrochaites* accession from Peru. LYC4943 was symptomless and tested virus-free by ELISA for more than 15 weeks after inoculation.

### Determination of TBRFV infection by ELISA

Infection was determined by ELISA. One apical leaf (fully expanded) of every plant was collected. Leaves were crushed using a Type R302 D63N-472 machine (VECTOR aandrijftechniek B.V., Rotterdam, The Netherlands) and sap was collected by adding 2 mL of PBS-Tween buffer. 100 µL of the extract was used for ELISA with antibodies against ToMV (supplier Prime Diagnostics, Wageningen, The Netherlands). ELISA reading was done by measurement of absorption at 405 nm with a FLUOstar Galaxy apparatus. Plants that gave absorption values more than 1.5 times of the clean control plants were considered infected (susceptible).

### Bioassays and mapping of TBRFV resistance genomic sequence

The original LYC4943 (*S*. *habrochaites)* seed lot is segregating for the resistance. Nine different F1 families were sown for bioassay in order to identify the F1 families which are completely resistant (resistance is fixed) and which will be used for further backcrosses. Four F1 families germinated and were tested in bioassay. F1 plants created with LYC4943 plant 3 (90479-3) were selected for resistance phenotype to create two populations for mapping choosing the *S*. *lycopersicum* lines OT9 and OT1317 as backcross lines. Markers M1 to M42 (respectively SEQ ID No. 19 to SEQ ID No.102) have been used in the mapping are listed in Table 1.

298 plants (OT9 x 90479-3) x OT9) and 484 plants (OT1317 x 90479-3) = total of 782 plants were inoculated with the TBRVF isolate AE050. Two to three weeks after inoculation the TBRFV symptoms were present and phenotyping by eye was done. A clear segregation was observed between resistant (R) and susceptible plants (S) and resistant phenotypes could be linked with marker M1 (see Table 1) located on chromosome 8 at 2673609 bp on the reference genome SL2.40 (*S. lycopersicum*). 92 plants have been genotyped with 26 markers in order to flank the QTL (M2 to M27, see Table 1). Based on these results the resistance could be mapped between 53118984 bp and 57038544 bp on the reference genome SL2.40 (between M8 & M20, See Table 1 and Figure 1).

**Table 1.**

| **Primer name** | **Primer sequence** | **Pos. SL2.40** | **Pos. on Locus** | **SEQ ID No.** |
|---|---|---|---|---|
| M1_F | GGTACAACAATTGACCAAGG | 2672994 | | 19 |
| M1_R | GCTAATTAAAAAGGAACATCAGC | | | 20 |
| M2_F | GCTATGGCGGAGAAGTCAAG | 18124 | | 21 |
| M2_R | AGTCACCTCCATAGTAGACC | | | 22 |
| M3_F | GGATCCAAGTTGTGTTCGAAC | 881036 | | 23 |
| M3_R | CTTCTCATCAATGTATGTGATTTC | | | 24 |
| M4_F | TGTATAACACCTGGTGCTCC | 15384575 | | 25 |
| M4_R | CCATTTTCTGTTACAAAATTTCAG | | | 26 |
| M5_F | GCTTCCCAATTTATGCTGAAG | 47887679 | | 27 |
| M5_R | GAGCCTCCCACTATAGTAATC | | | 28 |
| M6_F | AGAATTATCATTTGCAGGATCG | 50957946 | | 29 |
| M6_R | CTATGGTTCGCATGTCATGC | | | 30 |
| M7_F | CACAACGGCAATATACCTTGC | 53082561 | | 31 |
| M7_R | TGGAAGTATTAGAAAGGTCCAG | | | 32 |
| M8_F | CCATTGAGAATAACTACTGTAC | 53118984 | | 33 |
| M8_R | CCACAGGATGACTAACTTGG | | | 34 |
| M9_F | TGCAGTATTGATCGCATCTTCTA | 53452252 | | 35 |
| M9_R | GTTTGTTGCTGCCCTCAAA | | | 36 |
| M10_F | TGATCAAGAATTTTGTTTTAGCATAGA | 55664335 | | 37 |
| M10_R | TAAAGCATCAATTTTGCATTGTCT | | | 38 |
| M11_F | TCGAAGACTAACAAAGTCCTTGTAGA | 55720872 | | 39 |
| M11_R | GACACTCCGGCAGTTCCTT | | | 40 |
| M12_F | TTCTTATGTGAAAAATTGGGTGG | 55776574 | | 41 |
| M12_R | ACTACGCAGTCCCACAGCTT | | | 42 |
| M13_F | TTGTTTGGTGGATCCATGTG | 56448988 | | 43 |
| M13_R | AGGGAAAGGGCAAGGATG | | | 44 |
| M14_F | GATCTACCAATGGCTATTCATC | 56781521 | | 45 |
| M14_R | GCAAAACTTAACCGGTCTAAG | | | 46 |
| M15_F | TCTCGATGGTTGATAATTTGTTC | 56874054 | | 47 |
| M15_R | GGAATCGATTAACACTGGTTC | | | 48 |
| M16_F | CATCTTATTGAAGCTCTGCTG | 56920720 | | 49 |
| M16_R | CAAACAGTCCCTATTCAACAC | | | 50 |
| M17_F | GGTCTTGCGCTAATCAAAAG | 56990004 | | 51 |
| M17_R | GCGTTGTGGTGAAAGTTTTATC | | | 52 |
| M18_F | CTTGTTTGGATGGTTGTCAC | 57003163 | | 53 |
| M18_R | CAACAAAAAATATACAATCCGTCC | | | 54 |
| M19_F | GAGATAGAAGGAAACTTACCG | 57024614 | | 55 |
| M19_R | CAATTATCCCCTCAGTTCTG | | | 56 |
| M20_F | TATGCCTGTCCCTGAAAAGG | 57038544 | | 57 |
| M20_R | AGGGTCTTGGATCAAATCTTGA | | | 58 |
| M21_F | TGTGGACTTGGAGTGGTATC | 57427631 | | 59 |
| M21_R | GTAGAAAGGGTAGGCATGTTC | | | 60 |
| M22_F | TACCAAAGCAAACACTGCCAC | 57441418 | | 61 |
| M22_R | AGCCACGAGATATATATTGGAG | | | 62 |
| M23_F | GATAAGACCGCCAATAACTAG | 60844273 | | 63 |
| M23_R | GTGATCTCCATGAGCAAATG | | | 64 |
| M24_F | TGAGTTGAGATGCTGTTCTAG | 61412883 | | 65 |
| M24_R | AGTCCACCAAGACTTAAAGAG | | | 66 |
| M25_F | GTCTGCCTTCTCTTGCATGC | 62277547 | | 67 |
| M25_R | GTTGCTCCAGACAGAATAAGC | | | 68 |
| M26_F | CATCGAAGAGATGTGTAGGG | 62418391 | | 69 |
| M26_R | TGCAGTTGAAGTAGACTTCAG | | | 70 |
| M27_F | TCAACGTTAGTGGTGATGCTAG | 62783214 | | 71 |
| M27_R | CAATTGCAGAAAGTGAAGCTG | | | 72 |
| M28_F | GTGGATTCAGTTAAACCAGAAC | 56924513 | 4076 | 73 |
| M28_R | GACATGTGGAACTTGACAAAAC | | | 74 |
| M29_F | GCGAGAGAAAAGATTCTCTAC | 56934501 | 12765 | 75 |
| M29_R | CATTCTTCACTCTCTCAAGATG | | | 76 |
| M30_F | CGTTTGGTGATCTGCCTTGTCTT | 56934846 | 13109 | 77 |
| M30_R | TCTTCTTGTAGGGAATCCAGAATC | | | 78 |
| M31_F | GTGTCCTGTGCTTGTTATTCC | 56935054 | 13317 | 79 |
| M31_R | CCTCAAACCTATTGCATCTGACA | | | 80 |
| M32_F | CGGCTCAGCGAGGAAGTGCAG | 56935849 | 14113 | 81 |
| M32_R | CGTTGACTGTTTTTCTTTATG | | | 82 |
| M33_F | GTAAGCTCCTTCATGTCAGC | 56941043 | 15893 | 83 |
| M33_R | CAAGTATTGTCTGCCGAGTAAC | | | 84 |
| M34_F | GCGTACAGACATATTTATGCAAC | 56942927 | 17777 | 85 |
| M34_R | GAACAGCTAAAAGTAAGAGCAC | | | 86 |
| M35_F | GTTCATGTGTGTTTATGGACC | 56943610 | 18416 | 87 |
| M35_R | CTTCACTAAATAAATAAGTGGTAG | | | 88 |
| M36_F | TATGGATTTGTGTCTCAGAAGA | 56944105 | 18912 | 89 |
| M36_R | TGTGGTCACCAAGTGGGTTTC | | | 90 |
| M37_F | GTCTTCCAGAGCAGTTATGCAAG | 56945167 | 19974 | 91 |
| M37_R | TGAGACTGCTAAGTTGACTTGTTTG | | | 92 |
| M38_F | GTACACCAAATCACAGACATCG | 56958371 | 101133 | 93 |
| M38_R | CCCAATTTGGTTTGTGTTGGAC | | | 94 |
| M39_F | GAAATTCCTTGCCTCCTCTC | 56961307 | 104063 | 95 |
| M39_R | GTGGAAGCCATAGTGTACAAG | | | 96 |
| M40_F | CATATTATACAGTGAAAGCTTTG | 56965103 | 107926 | 97 |
| M40_R | GAATTGCAGTTCACTTGCTTC | | | 98 |
| M41_F | CCACAAAGCTAAAAAGGGATTG | 56969685 | 112529 | 99 |
| M41_R | TCCATGTGAGTTTTGTGTGTG | | | 100 |
| M42_F | GCCACATAAATTACATATAGCTG | 56981278 | 125792 | 101 |
| M42_R | GAACTATTCAACAAGCATAATAC | | | 102 |
| M-SEQ 10_F | GTCTTACAATAGTAAAATGCGCAG | | 36480 | 105 |
| M-SEQ 10_R | GCGGTTCGTTGATATTCCAAC | | | 106 |
| M-SEQ 11_1F | AGCGAAAGCGGAAGGAGTAC | | 48748 | 107 |
| M-SEQ 11_1R | TGTGGTGAGTAAGCAATGAATC | | | 108 |
| M-SEQ 11_2F | GTGTATAATTCGCCAGAATATACGG | | 52303 | 109 |
| M-SEQ 11_2R | CGTTTAGATAATTGTATATTACACATATG | | | 110 |
| M-SEQ 14_F | CAAATTATTACTTATGTTGTGATTTG | | 77410 | 111 |
| M-SEQ 14_R | ATTAAGCCATGATACACAAATTAC | | | 112 |

The whole population of 782 plants have been genotyped with the flanking markers M8 & M20 in order to find the recombinant plants for further fine mapping. This resulted in 21 recombinant plants (See Figure 1). These 21 recombinant plants have been selected and genotyped with 11 markers M9 to M19 in order to further fine map the region (Table 1). The resistance could be fine mapped between 56920720 and 56990004 (marker M16 and M17) on the reference genome SL2.40.

Sequencing the resistant LYC4943 region using Oxford Nanopore sequencing technology resulted in a locus of 133.515 bp. The 21 recombinant plants have been genotyped with extra markers in this specific locus (M28 to M42) of LYC4943. Based on the recombinant plants, plant 594 and 608, it was determined that the resistant region is located between positions 56941043 and 56958371, based on the reference genome SL2.40, corresponding with positions between 15.893 and 101.133 on the LYC4943 locus (between M33 and M38, see Figure 1).

Based on the fine mapping, the size and location of the genomic sequence that is harbouring the TBRFV resistance is determined to be between markers M33 and M38 and is approximately 68.000 bp larger compared to the SL2.40 reference genome of *S*. *lycopersicum* (85.240 bp vs. 17.328 bp, respectively). It is therefore highly likely that one or more genes are located within this region, indicated in figure 1 as "TBRFV region", providing the TBRFV resistance and is indicated as SEQ ID No.3 in this application. Based on the reference genome SL2.40 and *in silico* prediction analysis (ITAG 2.3), at least one gene is located in the fine mapped region that encodes for a CC-NBS-LRR resistance protein. Blasting the fine mapped TBRFV region against the database of National Center for Biotechnology Information (NCBI), resulted in seven genomic fragments of which five do have homology with NBS-LRR resistance proteins (SEQ ID No.8, No.9, No.10, No.11 and No.14) and two do have homology with LRR receptor-like serine/threonine-protein kinases (SEQ ID No.12 and SEQ ID No.13).

Next, further finemapping was performed and a recombinant selection has been performed by genotyping 668 BC2 plants ((OT9 x 90479-3) x OT9 x OT9) with M33 and M38 in order to identify recombinant plants in the TBRFV region, which resulting in three plants 15321-02, 15321-03 and 15321-07 (see Figure 4). These three plants were tested for resistance by inoculation with TBRFV isolate AE50. Approximately three weeks after TBRFV inoculation the plants were phenotyped by observation, and ELISA and qPCR was performed to monitor virus infection. The recombinant plants have been genotyped with markers (M-SEQ 10, M-SEQ 11-1, M-SEQ 11-2, and M-SEQ 14, respectively SEQ ID No. 105 to SEQ ID No. 112) covering the TBRFV locus and were specifically designed to eliminate candidate genes in the TBRFV locus. This approach provides insight in which of the candidate genomic sequences SEQ ID No.1 to SEQ ID No.18 specifically provides resistance to TBRFV. Based on the recombinant plants and phenotyping by disease tests, ELISA and qPCR, we concluded that the gene conferring resistance is encoded by genomic sequence of SEQ ID No 14, more specifically the coding DNA sequence of SEQ ID No. 115 encoding the protein of SEQ ID No. 116.

### Validation Tm0, Tm1 & Tm2 strain resistance in plant comprising the TBRFV resistance locus

A tomato plant (*S*. *lycopersicum*) comprising the TBRFV resistance locus (SEQ ID No. 1) was tested for resistance against the Tm0, 1 and 2 strains. The presence of the TBRFV resistance locus was determined by markers M16, M17 and M33. It was furthermore confirmed that the plant does not contain the *Tm2²* gene (is a known gene that provides resistance against Tm0, 1 and 2 strains). In some case the plant did contain the *Tm1* resistance gene. As a control plants were selected that did not contain the TBRFV resistance locus.

Eight plants (See Table 2, 1 to 8) of which six plants comprise the TBRFV resistance locus (heterozygous), and two plants (7 and 8) do not have the TBRFV resistance locus have been inoculated with the Tm0 isolate. Eight plants (See Table 2, 9 to 16) of which six plants comprise the TBRFV resistance locus (heterozygous), and two plants (15 and 16) do not have the TBRFV resistance locus, have been inoculated with the Tm-1 isolate. Eight plants (See Table 2, 17 to 28) of which four plants comprise the TBRFV resistance locus (two homozygous 17, 18 + two heterozygous 19, 20), and four plants not have the TBRFV resistance locus have been inoculated with the Tm2 isolate. As control the susceptible cultivated tomato line OT95 was also inoculated with all three strains.

First symptoms were typically observed after 12-14 days post-inoculation. Plants were categorized as Resistant (R) when no mosaic pattern symptoms on leaves were observed; plants displaying any of the symptoms on leaves were categorized as Susceptible (S). The phenotype of every single plant has been compared with the TBRFV genotype. Results are summarized in table 2 below.

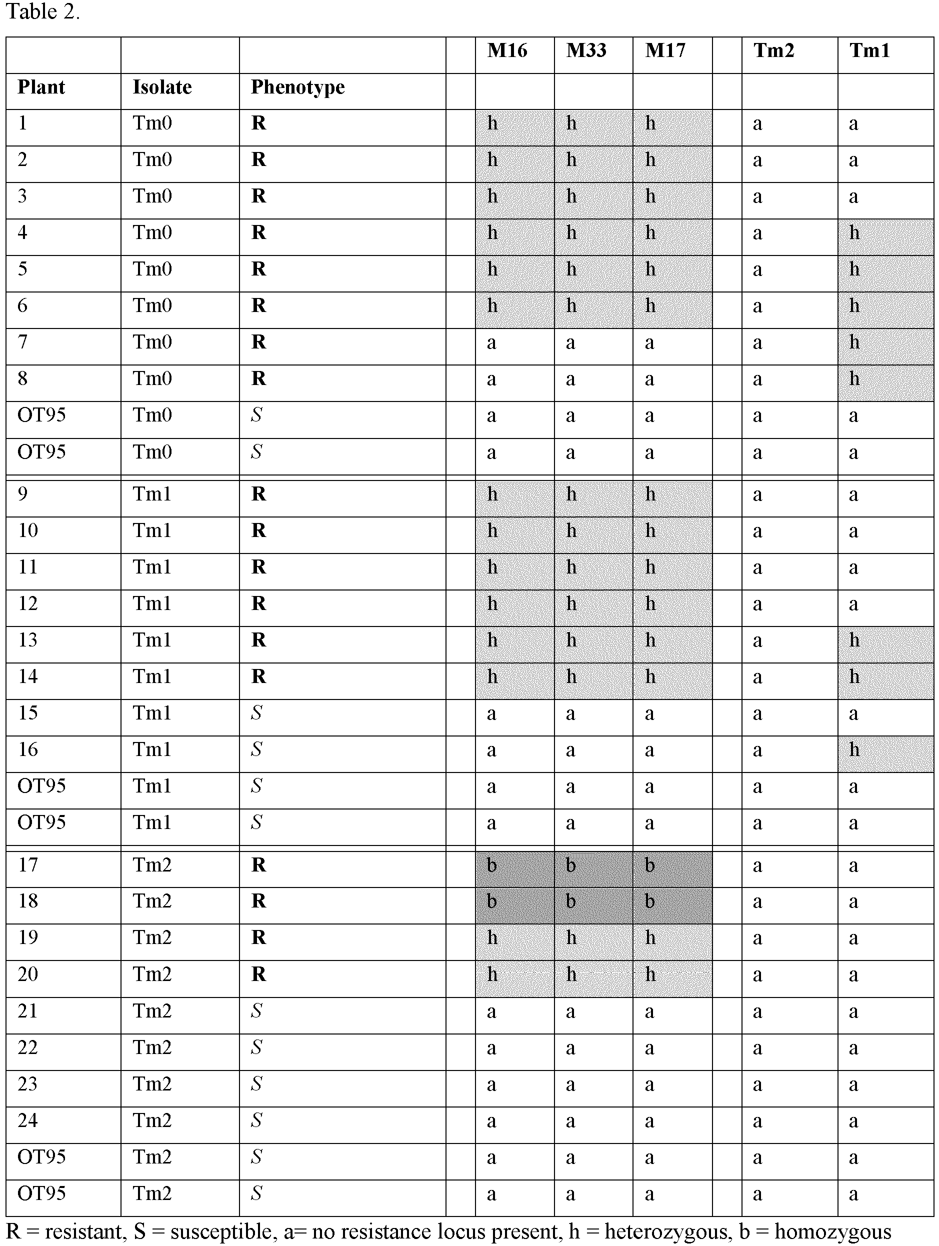

### Result Tm0

All plants that contained the TBRFV resistance locus are resistant. Plants 7 and 8, did not contain the TBRFV resistance locus but were also resistant. A reason that could explain the results is that the *Tm1* gene is causing the resistance to ToMV isolate Tm-0. In addition, plant 1, 2 and 3, did not contain the *Tm1* gene, but did contain the TBRFV resistance locus, showed to be resistant.

### Result Tm1

The resistant phenotypes are linked with the TBRFV genotypes, providing resistance against ToMV isolate Tm-1.

### Result Tm2

The resistant phenotypes (hetero-, homozygous) are linked with the TBRFV genotypes, providing resistance against ToMV isolate Tm-2.

### Determination of TBRFV infection in tomato (S. lycopersicum) by qPCR

Tomato plants comprising the TBRFV resistance locus (heterozygous or homozygous) and plants not containing this region have been selected for TBRFV bioassay using markers (M16 and M17). Plants were infected with TBRFV and the susceptible tomato line OT9 has been included as control (OT9 non-infected and infected).

After 3 weeks of inoculation, one leaf from the top of the plant of every single plant was collected in a 2 ml tube which contain an 6.35 mm metal bullet. The tube was frozen in liquid nitrogen. The tubes were shaken with high speed to pulverize the plant material. After spin down the tube, the standard RNA extraction using Macherey-Nagel^{™} NucleoSpin^{™} RNA Plant was carried out. RNA concentration was measured using DropSense 96 (trinean) and was diluted to a concentration of 100 ng/ul. 900 ng have been used for cDNA synthesis using M-MLV Reverse Transcriptase (Invitrogen). 10 ng cDNA was used for Real-time PCR using LC green as Intercalating dye. Two primer combinations for amplifying the TBRFV strain were used, see Table 3 (SEQ ID No.103 and SEQ ID No.104, respectively).

**Table 3.**

| **qPCR primer name** | **Sequence** |
|---|---|
| TBRFV-3 Fw | ACCGTTCAACGGCAATTTAGC |
| TBRFV-3 Rev | CCTATACACCTTAAAACCACTG |

The more viral RNA present in the samples the lower the Ct value in the qPCR, since less PCR cycles are required to amplify the cDNA (of the viral RNA) and pick up a signal. The control sample (OT9 uninfected) showed a Ct value of between 35 and 40 cycles and the infected control sample (OT9 infected) showed a Ct value between 20 and 25. Therefore plants that show a Ct value above 30 cycles, preferably around 35 cycles were considered resistant, whereas plants that show a Ct value below 30 were considered susceptible (see Figure 2).

Tomato plants comprising the TBRFV resistance locus, homozygous (B) or heterozygous (H) all have a Ct value above 30 cycles and can be considered as resistant. The results are showing that the resistance is dominant. Plants that did not comprise the TBRFV resistance locus (A) showed a Ct value of between 20 and 25, indicating that the plant was susceptible to TBRFV infection.

### Sequencing of genomic sequence of resistant tomato plant

Genomic DNA was isolated from a resistant plant (*S.lycopersicum*) comprising the TBRFV resistance locus, according to the protocol as published on 27 April 2018 in Nature, Protocol Exchange (2018), Rachael Workman *et al,. "High Molecular Weight DNA Extraction from Recalcitrant Plant Species for Third Generation Sequencing".* The sequencing libraries were prepared using the PCR free, no multiplex, DNA Ligation Sequencing Kit-Promethion (SQK-LSK109). The isolation procedure resulted in high quality sequencing libraries to be used in the Oxford Nanopore system for sequencing (ONT sequencing). Promethion Flowcell Packs (3000 pore / flowcell) version R9.4.1. were used for sequencing.

Furthermore, to further resolve the TBRFV locus and identify the gene providing the TBRFV resistance, we performed ONT sequencing on a resistant line (LYC4943). Sequencing of the entire transcript isoforms of the resistant LYC4943 line was done using the Iso-Seq analysis application (Pacific Biosciences of California, PacBio). This resulted in only one candidate resistance transcript/gene located in region between markers M33 and M38, more specifically the TBRFV resistance gene of SEQ ID No. 115. This transcript was predecited to encode for a CC-NBS-LRR resistant protein of SEQ ID No. 116.

### Gene validation using VIGS

To confirm that the TBRFV resistance gene (SEQ ID No 115) is indeed the gene conferring resistance to TBRFV, a Virus Induced Gene Silencing (VIGS) analysis was performed. Tobacco rattle virus (TRV)-derived VIGS vectors have been abundantly described to study gene function in plants such as Arabidopsis thaliana, Nicotiana benthamiana, Lycopersicon esculentum and other plants (see for example Huang C, Qian Y, Li Z, Zhou X.: Virus-induced gene silencing and its application in plant functional genomics. Sci China Life Sci. 2012;55(2):99-108).

As such, two VIGS constructs were developed (Table 4), one construct VIGS-01a to specifically target SEQ ID No 115 and a control construct VIGS-01b that targets SEQ ID No. 7, i.e. a sequence also located within the previously identified TBRFV locus.

**Table 4.**

| **VIGS construct** | **Sequence** |
|---|---|
| VIGS-01a (SEQ ID No. 113) | GGAAGATTTTAATGAAAAGAGGTTGATAAAGAAAATTGTAGAAT CTATTGAAGAAAAGTCACTTGGTGACATGGACTTGGCTCCACTTC AAAAGAAGCTTCAGGACTTGCTGAATGGAAAAAAATATTTGCTTG TCTTAGATGATGTTTGGAATGAAGATCAAGATAAGTGGGCTAAGT TAAGACAAGTCTTGAAGGCTGGAGCAAGTGGTGCTTATGTTCTAA CCACTACC |
| VIGS-01b (SEQ ID No. 114) | AGAAGATTTTGATGAGAAGAAGTTGATAAAGGCAATTGTTGAATC TATCGAAGGAAACCCACTTGGTGACCACATGGATTTGGCTCCACT TCAAAAGAAGCTTCAGGACATGTTGAATGGAAAGAGATACTTTCT CGTTTTGGATGATGTTTGGAATGAAAATCAAGAAAAGTGGGATAA GATAAAAGCAGTCTTAGAGGTTGGAGCACGAGGTGCTTCTGTTCT AACCACCACT |

The VIGS fragments were synthesized (IDT, gBlocks) and subsequently cloned into a TRV vector. The DNA sequences were confirmed by Sanger sequencing. The vector contains all sequences encoding for proteins that are required for a functional TRV particles including the target sequences. The VIGS vectors including the VIGS-01a and VIGS-01b constructs were transformed into Agrobacterium tumefaciens strain GV3101 and used in VIGS experiments to reduce endogenous mRNA levels in tomato plants used in this experiment. A homozygous TBRFV resistant line (15322-04) as well as a susceptible control line (OT9) were used in the VIGS experiment, in which plants were agrobacterium infiltrated at seedling stage (cotyledons) followed by TBRFV isolate E50 inoculation three weeks after agrobacterium infiltration. Two weeks after TBRFV inoculation the individual plants were phenotyped by ELISA and qPCR and revealed that susceptibility was found in resistant plants infiltrated with construct VIGS-01a whereas no susceptibility has been detected in resistant plants infiltrated using construct VIGS-01b. Results of the ELISA and qPCR are shown in Figures 5 and 6, and results have been summirized in Table 5.

**Table 5.**

| **Plants** | **# plants** | **VIGS-construct** | **TBRFV infection** | **# S plants** | **# R plants** |
|---|---|---|---|---|---|
| R line 15322-04 | 7 | VIGS-01a | Yes | 7 | 0 |
| R line 15322-04 | 6 | VIGS-01b | Yes | 0 | 6 |
| R line 15322-04 | 10 | No | Yes | 0 | 10 |
| S line OT9 | 6 | No | Yes | 6 | 0 |

The OT9 line all plants were susceptible, as expected. The R line which was shown earlier to be fully resistant became susceptible to TBRFV in case the suspected TBRFV resistance gene was silenced using the VIGS-01a construct designed to specifically target this gene, whereas silencing using the VIGS-01b construct (controle construct) did not result in any susceptibility of the plants tested. Based on these results it can be concluded that gene SEQ ID No 115 is the conferring resistance to TBRFV.

## Claims

1. Resistance gene for providing resistance to a Tobamovirus in a *S*. *lycopersicum* plant, wherein said resistance gene is represented by a coding sequence having at least 95% nucleotide sequence identity with SEQ ID No.115, wherein the Tobamovirus is TBRFV.

2. Genomic sequence for providing resistance to a Tobamovirus in a *S*. *lycopersicum* plant, wherein the genomic sequence is SEQ ID No.14, or having at least 95% sequence identity with SEQ ID No 14 , wherein the Tobamovirus is TBRFV.

3. A method for selecting a plant of the S. lycopersicum species that is resistant to Tobamovirus, wherein said selection comprises establishing the presence of a resistance gene, genomic sequence according claim 1 or 2, preferably said resistance gene.

4. Method according to claim 3, wherein establishing the presence of the resistance gene, resistance conferring genomic sequence or resistance locus is performed by one or more markers selected from the group consisting of SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, and SEQ ID No: 94, SEQ ID No. 105, SEQ ID No. 106, SEQ ID No. 107, SEQ ID No. 108, SEQ ID No. 109, SEQ ID No. 110, SEQ ID No. 111, and SEQ ID No. 112, preferably SEQ ID No. 105, SEQ ID No. 106, SEQ ID No. 107, SEQ ID No. 108, SEQ ID No. 109, SEQ ID No. 110, SEQ ID No. 111, and SEQ ID No. 112.

5. Use of a marker for establishing the presence of a TBRFV resistance gene, resistance conferring genomic sequence according to claim 1 or 2 in a *S*. *lycopersicum* plant, wherein the marker is one or more selected from the group consisting of SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, and SEQ ID No: 94, SEQ ID No. 105, SEQ ID No. 106, SEQ ID No. 107, SEQ ID No. 108, SEQ ID No. 109, SEQ ID No. 110, SEQ ID No. 111, and SEQ ID No. 112, preferably SEQ ID No. 105, SEQ ID No. 106, SEQ ID No. 107, SEQ ID No. 108, SEQ ID No. 109, SEQ ID No. 110, SEQ ID No. 111, and SEQ ID No. 112.

## Patentansprüche

1. Resistenzgen zum Bereitstellen einer Resistenz gegen ein Tobamovirus in einer S. lycopersicum-Pflanze, wobei das Resistenzgen durch eine codierende Sequenz dargestellt wird, die mindestens zu 95 % eine Nukleotidsequenzidentität mit SEQ ID No. 115 aufweist, wobei das Tobamovirus TBRFV ist.

2. Genomsequenz zum Bereitstellen einer Resistenz gegen ein Tobamovirus in einer S. lycopersicum-Pflanze, wobei die Genomsequenz SEQ ID No. 14 ist oder mindestens zu 95 % eine Sequenzidentität mit SEQ ID No 14 aufweist, wobei das Tobamovirus TBRFV ist.

3. Verfahren zum Auswählen einer Pflanze der Art S. lycopersicum, die gegen Tobamovirus resistent ist, wobei die Auswahl ein Nachweisen des Vorhandenseins eines Resistenzgens oder einer Genomsequenz nach Anspruch 1 oder 2, vorzugsweise das Resistenzgen, umfasst.

4. Verfahren nach Anspruch 3, wobei das Nachweisen des Vorhandenseins des Resistenzgens, der Resistenz verleihenden Genomsequenz oder des Resistenzlocus durch einen oder mehrere Marker durchgeführt wird, die ausgewählt sind aus der Gruppe bestehend aus SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93 und SEQ ID No: 94, SEQ ID No. 105, SEQ ID No. 106, SEQ ID No. 107, SEQ ID No. 108, SEQ ID No. 109, SEQ ID No. 110, SEQ ID No. 111 und SEQ ID No. 112, vorzugsweise SEQ ID No. 105, SEQ ID No. 106, SEQ ID No. 107, SEQ ID No. 108, SEQ ID No. 109, SEQ ID No. 110, SEQ ID No. 111 und SEQ ID No. 112.

5. Verwendung eines Markers zum Nachweisen des Vorhandenseins eines TBRFV-Resistenzgens, einer Resistenz verleihende Genomsequenz nach Anspruch 1 oder 2 in einer S. lycopersicum-Pflanze, wobei der Marker einer oder mehrere ist, ausgewählt aus der Gruppe bestehend aus SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93 und SEQ ID No: 94, SEQ ID No. 105, SEQ ID No. 106, SEQ ID No. 107, SEQ ID No. 108, SEQ ID No. 109, SEQ ID No. 110, SEQ ID No. 111 und SEQ ID No. 112, vorzugsweise SEQ ID No. 105, SEQ ID No. 106, SEQ ID No. 107, SEQ ID No. 108, SEQ ID No. 109, SEQ ID No. 110, SEQ ID No. 111 und SEQ ID No. 112.

## Revendications

1. Gène de résistance permettant de fournir une résistance à un Tobamovirus dans une plante *S. lycopersicum,* dans lequel ledit gène de résistance est représenté par une séquence codante ayant au moins 95 % d'identité de séquence nucléotidique avec SEQ ID No.115, dans lequel le Tobamovirus est TBRFV.

2. Séquence génomique permettant de fournir une résistance à un Tobamovirus dans une plante *S. lycopersicum,* dans laquelle la séquence génomique est SEQ ID No.14, ou a au moins 95 % d'identité de séquence avec SEQ ID No.14, dans laquelle le Tobamovirus est TBRFV.

3. Procédé permettant de sélectionner une plante de l'espèce S. lycopersicum qui est résistante au Tobamovirus, dans lequel ladite sélection comprend l'établissement de la présence d'un gène de résistance, d'une séquence génomique selon la revendication 1 ou 2, de préférence ledit gène de résistance.

4. Procédé selon la revendication 3, dans lequel l'établissement de la présence du gène de résistance, de la séquence génomique conférant la résistance ou d'un locus de résistance est mis en œuvre par un ou plusieurs marqueurs choisis dans le groupe constitué par SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, SEQ ID No: 94, SEQ ID No.105, SEQ ID No.106, SEQ ID No.107, SEQ ID No.108, SEQ ID No.109, SEQ ID No.110, SEQ ID No.111, et SEQ ID No.112, de préférence SEQ ID No.105, SEQ ID No.106, SEQ ID No.107, SEQ ID No.108, SEQ ID No.109, SEQ ID No.110, SEQ ID No.111, et SEQ ID No.112.

5. Utilisation d'un marqueur pour établir la présence d'un gène de résistance au TBRFV, d'une séquence génomique conférant une résistance selon la revendication 1 ou 2 dans une plante *S. lycopersicum,* dans laquelle le marqueur est un ou plusieurs marqueurs choisis dans le groupe constitué par SEQ ID No: 83, SEQ ID No: 84, SEQ ID No: 85, SEQ ID No: 86, SEQ ID No: 87, SEQ ID No: 88, SEQ ID No: 89, SEQ ID No: 90, SEQ ID No: 91, SEQ ID No: 92, SEQ ID No: 93, SEQ ID No: 94, SEQ ID No.105, SEQ ID No.106, SEQ ID No.107, SEQ ID No.108, SEQ ID No.109, SEQ ID No.110, SEQ ID No.111, et SEQ ID No.112, de préférence SEQ ID No.105, SEQ ID No.106, SEQ ID No.107, SEQ ID No.108, SEQ ID No.109, SEQ ID No.110, SEQ ID No.111, et SEQ ID No.112.
